# EUROPEAN PATENT APPLICATION

(11) **EP 4 273 303 A1**
(43) Date of publication of application: **08.11.2023**
(21) Application number: 22171849.7
(22) Date of filing: 05.05.2022
(51) Int. Cl.: C25D 3/56, C25D 21/14, C25D 21/18, C25D 21/20

(54) **METHOD FOR DEPOSITING A ZINC-NICKEL ALLOY ON A SUBSTRATE, AN AQUEOUS ZINC-NICKEL DEPOSITION BATH, A BRIGHTENING AGENT AND USE THEREOF**

(71) Applicant: Atotech Deutschland GmbH & Co. KG, 10553 Berlin (DE)
(72) Inventor: Kühne, Sebastian, 10553 Berlin (DE); Laudien, Ulrich, 10553 Berlin (DE); Kaczmarek, Michal, 10553 Berlin (DE); Dziurun, Marta, 10553 Berlin (DE); Richter, Richard, 10553 Berlin (DE); Brunner, Heiko, 10553 Berlin (DE); Leonhard, Steven, 10553 Berlin (DE); Freese, Thomas, 10553 Berlin (DE)
(74) Representative: Atotech Deutschland GmbH & Co. KG

(57) **Abstract**

The present invention relates to a method for depositing a zinc-nickel alloy on a substrate, particularly to a method for electrolytically depositing a zinc-nickel alloy on a substrate, wherein a zinc-nickel deposition bath is utilized as a catholyte comprising at least one brightening agent, wherein the at least one brightening agent has a source which is substantially free of, preferably does not comprise, halogen anions. The present invention furthermore relates to a respective aqueous zinc-nickel deposition bath, a respective brightening agent, and a respective use of said brightening agent for replenishing in an aqueous zinc-nickel deposition bath.

## Description

### Field of the Invention

The present invention relates to a method for depositing a zinc-nickel alloy on a substrate, particularly to a method for electrolytically depositing a zinc-nickel alloy on a substrate, wherein a zinc-nickel deposition bath is utilized as a catholyte comprising at least one brightening agent, wherein the at least one brightening agent has a source which is substantially free of, preferably does not comprise, halogen anions. The present invention furthermore relates to a respective aqueous zinc-nickel deposition bath, a respective brightening agent, and a respective use of said brightening agent for replenishing in an aqueous zinc-nickel deposition bath.

### Background of the Invention

Depositing a metal alloy, sometimes also referred to as a coating or protective coating, on other metals or metal-coated plastics (typically referred to as substrates) is a well-established technique to increase the corrosion resistance of such substrates. The deposition is usually carried out electrolytically using anodes and the substrate as a cathode in a respective electrolyte (typically known as deposition or plating bath). A well-known example is the galvanization of metal substrates with a zinc-nickel alloy.

It has a number of advantages to separate the electrolyte by means of a separator, e.g. a semipermeable membrane, into a catholyte which is the electrolyte in a respective cathode compartment, and an anolyte which is the electrolyte in a respective anode compartment. Often the anolyte is different from the catholyte. By applying an electrical potential, a current is flowing via the anolyte through the separator to the catholyte to electrolytically deposit the metal alloy on the substrate.

US 2011/031127 A1 discloses an alkaline electroplating bath for plating zinc-nickel coatings, having an anode and a cathode, wherein the anode is separated from the alkaline electrolyte by an ion exchange membrane.

WO 2021/123129 A1 discloses a method for depositing a zinc-nickel alloy on a substrate, wherein a deposition bath is utilized comprising at least one complexing agent for nickel ions, wherein the nickel ions have a source which is substantially free of, preferably does not comprise, complexing agents.

As a matter of fact, such methods are very efficient and provide excellent corrosion protection.

Typically, zinc-nickel deposition baths are often used continuously for an extended period of time, for example for month or even years. It is commonly known that a high throughput requires a continuous replenishment of zinc and nickel ions as well as organic compounds such as complexing agents and brightening agents. A certain amount thereof is simply incorporated in the zinc alloy layer, in particular zinc ions, nickel ions, and brightening agents. Another amount is typically lost via drag-out. Another portion is decomposed, e.g. anodically, and remains present in a decomposed form. Typically, decomposition is often neglectable because drag-out does not only reduce the concentration of desired compounds but also of undesired decomposition products. The desired compounds are therefore simply replenished.

However, as a matter of fact, such prior art methods also produce a significant amount of wastewater, typically contaminated with nickel ions, zinc ions, complexing agents, brightening agents (all not desired to be wasted), and decomposition products (desired to be removed from the process) of at least some of them. Since environmental demands, sustainability, and wastewater demands are more and more important topics, there is a constant requirement to provide methods which are more environmentally friendly, more sustainable, and particularly more carefully dealing with the resource water.

WO 2021/123129 A1 therefore suggests a method carried out in a loop to avoid or at least drastically reduce wastewater and wastewater contamination by providing a loop-for zinc nickel deposition. However, such a loop shows drastic influence on the concentration level of involved compounds, e.g. complexing agents, nickel and zinc ions, respectively, if wastewater is constantly recycled in the loop. Typically, further insight is required into such new developments, which very often need long-time observations to identify surprising and unpredictable effects. Since no significant drag out takes place in such a method, decomposition products and undesired compounds start to accumulate. As a matter of fact, this must be prevented. Very often no investigations were so far possible to identify under what circumstances they become detrimental and how to remove or avoid them.

### Objectives of the present Invention

It was therefore the objective of the present invention to provide a further improved method for depositing a zinc-nickel alloy on a substrate, in particular a respective method carried out in a loop with a high level of recycling involved (i.e. in particular wastewater recycling). It was particularly the objective of the present invention to provide a respective method with increased sustainability by further increasing operating time without compromising the deposition quality, corrosion resistance of galvanized substrates, and maintaining a stable deposition environment over a longer operation time, including a further increased lifetime of a respective deposition bath and avoiding harm to the respective plating equipment.

### Summary of the Invention

The objectives mentioned above are solved by a method for depositing a zinc-nickel alloy on a substrate, the method comprising the steps:
(a) providing the substrate,
   (a-1) optionally, pre-rinsing the substrate with water in a pre-rinsing compartment such that a pre-rinsed substrate and pre-rinse water is obtained,
(b) providing an aqueous zinc-nickel deposition bath as a catholyte in a deposition compartment, wherein
   - the deposition compartment comprises at least one anode with an anolyte, and
   - the anolyte is separated from the catholyte by at least one membrane, and the catholyte comprises
      (i) nickel ions,
      (ii) at least one brightening agent, and
      (iii) zinc ions,
(c) contacting the substrate or the pre-rinsed substrate with the catholyte in the deposition compartment such that the zinc-nickel alloy is electrolytically deposited onto the substrate and thereby obtaining a zinc-nickel coated substrate, wherein
   after step (c) the at least one brightening agent has a lower concentration than before step (c), characterized in that the method comprises after a step (c), step
(d) adding directly or indirectly a brightening agent source to the catholyte, said source being substantially free of, preferably not comprising, halogen anions.

Own long-time observations have shown that a method for depositing a zinc-nickel alloy including an improved sustainability, preferably by means of a loop or most preferably a closed-loop, is comparatively sensitive to even small amounts of halogen ions, most preferably chloride ions. Although it was believed that relatively small amounts of chloride ions can be co-removed together with e.g. sulfate ions in a precipitation step as disclosed in WO 2021/123129 A1, these own experiments have now shown that even small amounts of halogen anions are undesired, even if a nickel ion source and a zinc ion source is used free of chloride ions. These own observations have revealed that a significant contributor to undesired halogen contamination, particularly chloride ion contamination, is the brightening agent; either directly in form of its counter anion or indirectly in form of insufficient purification after synthesis. Since a brightening agent is constantly consumed during the deposition process, typical counterions thereof such as halogen anions, particularly chloride ions, start to accumulate. Although such an accumulation takes place on a relatively low concentration level, it was surprisingly noted that even such a low concentration level leads to undesired effects on equipment parts over time. It was particularly observed that an undesired corrosion sometimes occur e.g. at equipment parts comprising stainless steel and/or at said at least one membrane due to the anodic formation of hypochlorite ions. A comparatively high chloride ion concentration also shows sometimes undesired effects on the stability of the at least one membrane separating anolyte and catholyte.

The method of the present invention solves the above defined objective and allows a loop (also named "operated in a loop"), most preferably even a closed-loop which is most preferred in the context of the present invention, for an even increased (i.e. prolonged) operation time. Own experiments have shown that said corrosion and/or gas formation is drastically reduced or even fully prevented by carrying out the method of the present invention and by keeping the brightening agent source substantially free of halogen anions, most preferably at least free from chloride ions. Furthermore, the method of the present invention allows a precipitation of e.g. sulfate ions independently from a halogen ion concentration.

Furthermore, the method of the present invention does not impair the excellent corrosion resistance of zinc-nickel galvanized substrates. Furthermore, due to the at least one membrane, the method of the present invention prevents the formation of detrimental cyanide and oxalate anions. This dramatically reduces the effort for wastewater treatment and is a significant pre-requisite for a loop, preferably a closed-loop, operation.

### Detailed Description of the Invention

In the context of the present invention, the term "at least one", "one or more than one", and/or "one or more" denotes (and is exchangeable with) "one, two, three or more than three".

Furthermore, in the context of the present invention, the zinc-nickel alloy deposited on the substrate is preferably a zinc-nickel alloy layer. It is preferably also called a zinc-nickel galvanization layer. Thus, the method of the present invention is preferably a galvanization method. This means that in step (c) preferably a current is applied.

Also, in the context of the present invention, "brightening agent source" includes the brightening agent itself, possible counter ions thereof, as well as leftovers of educt compounds and synthesis products (e.g. leaving groups).

### Step (a): providing the substrate:

In step (a) the substrate is provided.

Preferred is a method of the present invention, wherein the substrate is a metallic substrate, preferably comprising iron, more preferably the substrate is an iron substrate, most preferably a steel substrate.

Preferred is a method of the present invention, wherein the substrate comprises substrates for
barrel applications,
   preferably fasteners, most preferably comprising at least screws, nuts, bolts, and washers, and/or
rack-applications,
   preferably comprising fittings, flat-, L-, or U-shaped profiles, and/or pipes, most preferably locker components, window fittings, door mountings, and furniture fittings, and/or pressure pipes (preferably brake pipes and/or fuel pipes).

Preferably, the substrate does not comprise or is not cast iron. However, in some rare cases a method of the present invention is preferred, wherein the substrate does comprise or is cast iron.

### Step (a-1): pre-rinsing:

In step (a-1) the substrate is optionally pre-rinsed with water in a pre-rinsing compartment such that a pre-rinsed substrate and pre-rinse water is obtained. Such a pre-rinsing is preferred. This step is preferably carried out after step (a) and prior to step (c).

By pre-rinsing the substrate in the pre-rinsing compartment, potential contaminations and/or impurities on the substrate are removed before transferring the substrate into the deposition compartment. Preferably, the pre-rinsing in the pre-rinsing compartment is carried out by means of a pre-rinsing solution. Preferably the pre-rinsing solution is aqueous and preferably comprises a hydroxide, preferably a metal hydroxide, even more preferably an alkali metal hydroxide, most preferably sodium hydroxide. The pre-rinsing solution is preferably alkaline. Most preferably the pre-rinsing solution is substantially free of, preferably does not comprise, an organic solvent. Most preferably, the only solvent is water.

A method of the present invention is preferred, wherein the pre-rinse water is at least partly recycled and re-used, most preferably re-used within the method of the present invention.

### Step (b): providing the aqueous zinc-nickel deposition bath:

In step (b), the aqueous zinc-nickel deposition bath is provided as the catholyte in the deposition compartment. Thus, utilized in the method of the present invention, the aqueous zinc-nickel deposition bath is the catholyte. Therefore, in the following, features regarding the aqueous zinc-nickel deposition bath preferably likewise apply to the catholyte.

A method of the present invention is preferred, wherein the aqueous zinc-nickel deposition bath comprises more than 50 vol.-% water, based on the total volume of the aqueous zinc-nickel deposition bath, more preferably 75 vol.-% or more, even more preferably 85 vol.-% or more, most preferably 92 vol.-% or more. Preferably, water is the only solvent in the aqueous zinc-nickel deposition bath. Preferably, the aqueous zinc-nickel deposition bath is substantially free of, preferably does not comprise, an organic solvent.

A method of the present invention is preferred, wherein the aqueous zinc-nickel deposition bath is alkaline, preferably has a pH in a range from 10 to 14, more preferably from 11 to 13.3, even more preferably from 11.5 to 13, yet even more preferably from 12 to 12.9, most preferably from 12.3 to 12.8. Generally, preferred is a pH range from 12 to 14, preferably from 12.3 to 13.5.

The aqueous zinc-nickel deposition bath comprises (i) nickel ions. A method of the present invention is preferred, wherein in the aqueous zinc-nickel deposition bath the nickel ions have a total concentration below 3 g/L, based on the total volume of the aqueous zinc-nickel deposition bath, preferably of 2.5 g/L or below, more preferably of 2 g/L or below (in each case with more than zero g/L; i.e. 0 g/L is excluded).

Generally, preferred is that the nickel ions have a total concentration ranging from 0.2 g/L to 2.8 g/L, based on the total volume of the aqueous zinc-nickel deposition bath, preferably from 0.4 g/L to 2.6 g/L, more preferably from 0.5 g/L to 2.5 g/L, most preferably from 0.6 g/L to 2.3 g/L.

More preferably, the nickel ions have a total concentration ranging from 0.4 g/L to 1.9 g/L, preferably from 0.6 g/L to 1.7 g/L, more preferably from 0.7 g/L to 1.6 g/L, even more preferably from 0.8 g/L to 1.5 g/L, most preferably from 0.9 g/L to 1.4 g/L. This is most preferred for barrel applications. However, for rack applications, preferably the total concentration ranges from 0.4 g/L to 2 g/L, based on the total volume of the aqueous zinc-nickel deposition bath, more preferably from 0.5 g/L to 1 g/L, most preferably from 0.5 g/L to 0.8 g/L.

The aqueous zinc-nickel deposition bath comprises (iii) zinc ions. A method of the present invention is preferred, wherein in the aqueous zinc-nickel deposition bath the zinc ions have a total concentration below 11 g/L, based on the total volume of the aqueous zinc-nickel deposition bath, preferably of 10 g/L or below, most preferably of 9 g/L or below.

Preferably, the zinc ions have a total concentration ranging from 5 g/L to 9 g/L, more preferably from 5.2 g/L to 8.5 g/L, even more preferably from 5.4 g/L to 8 g/L, yet even more preferably from 5.7 g/L to 7.5 g/L, most preferably from 5.9 g/L to 7.3 g/L. Generally preferred is that the zinc ions have a total concentration ranging from 6 g/L to 9 g/L.

Preferred is a method of the present invention, wherein a zinc ion source is added directly or indirectly to the aqueous zinc-nickel deposition bath, preferably indirectly via a mixing unit. The mixing unit preferably comprises a separated volume of the (preferably utilized) aqueous zinc-nickel deposition bath. More preferably, zinc ions are obtained by dissolving metallic zinc in sodium hydroxide to obtain zinc hydroxo complexes, which allows for an efficient stabilization of the zinc ions in the aqueous zinc-nickel deposition bath. This preferably applies as long as the method of the present invention is carried out. Thus, it is most preferably performed for replenishing the zinc ions. Preferably, also nickel ions are replenished by indirectly adding a nickel ion source via the mixing unit such that a thoroughly mixed composition is prepared before transferring it into the catholyte and aqueous zinc-nickel deposition bath, respectively.

The mixing unit is preferably a separate pre-treatment compartment, most preferably fluidically connected with the deposition compartment. It most preferably provides a homogenization and pre-solubilization, respectively, by thorough mixing.

Most preferably, the nickel ions are replenished by means of a nickel ion source comprising an inorganic nickel salt, more preferably comprising nickel sulfate, most preferably nickel sulfate hexahydrate, even most preferably without additional (preferably organic) complexing agents for nickel ions (see also text below).

A method of the present invention is preferred, wherein the nickel ion source does not comprise nitrate ions. This most preferably means that neither the nickel salt nor the source comprises nitrate ions. By excluding nitrate ions, the concentration of nitrate ions in the catholyte is low or even entirely prevented. In many cases nitrate is disturbing the entire electrolytic deposition and is highly undesired. Furthermore, the nickel ion source does not comprise halogen anions, in particular no chloride ions.

A method of the present invention is preferred, wherein the nickel ion source does not comprise an organic amine, preferably does not comprise any amine. This most preferably means that neither the nickel salt nor the source comprises an organic amine. Amines are typical complexing agents for nickel ions (about complexing agents for nickel ions see text below). It is therefore preferred that the source of nickel ions is substantially free of, preferably does not comprise, a complexing agent for nickel ions. Thus, a complexing agent for nickel ions is preferably monitored and controlled independently from the nickel ion source. This is most preferred in a loop and closed-loop, respectively.

Most preferred is a method of the present invention, wherein the nickel ions of the nickel ion source added to the catholyte to replenish nickel ions, are not complexed before being in contact with an alkaline environment, preferably an environment having a pH ranging from 10 to 14, more preferably from 11 to 13.3, even more preferably from 11.5 to 13, yet even more preferably from 12 to 12.9, most preferably from 12.3 to 12.8; or ranging from 12 to 14, preferably from 12.5 to 13.5. Preferably, the nickel ions of the nickel ion source added to the catholyte are preferably complexed for the first time when contacted with an alkaline environment, preferably an environment having a pH as defined above, which is most preferably the catholyte.

A method of the present invention is preferred, wherein the nickel ion source is an aqueous solution comprising water and a nickel salt dissolved therein, preferably a nickel salt as defined above as being preferred.

Advantageously, in the method of the present invention the above defined total concentrations for nickel and zinc ions are typically below concentrations commonly known and used in the art for zinc-nickel deposition. Since nickel and zinc ions are preferably recycled in the method of the present invention (i.e. indirectly by means of recycling water such as rinse water), no significant amounts of nickel and zinc ions, respectively, are wasted, thus, leading to a reduced overall total concentration in the aqueous zinc-nickel deposition bath. Thus, preferred is a method of the present invention, wherein at least a portion of the nickel ions and the zinc ions is circulating in a loop, wherein the loop comprises a rinse step. More preferred is a method of the present invention, wherein at least a portion of the nickel ions, the zinc ions, the at least one brightening agent, and complexing agents is circulating in a loop, wherein the loop comprises a rinse step.

### The at least one brightening agent:

In the method of the present invention, the aqueous zinc-nickel deposition bath comprises at least one brightening agent. The at least one brightening agent is preferably required to provide a sufficient brightness and gloss as well as to further improve the electrolytic deposition in step (c). Thus, it primarily contributes to an improved optical appearance.

Generally preferred is a method of the present invention, wherein in the aqueous zinc-nickel deposition bath the at least one brightening agent has a total concentration ranging from 5 mg/L to 1200 mg/L, based on the total volume of the aqueous zinc-nickel deposition bath, preferably ranging from 10 mg/L to 1000 mg/L, more preferably ranging from 20 mg/L to 800 mg/L, even more preferably ranging from 30 mg/L to 600 mg/L, most preferably ranging from 40 mg/L to 450 mg/L, even most preferably ranging from 45 mg/L to 400 mg/L. This most preferably applies to both barrel as well as rack applications.

In some cases, preferred is a method of the present invention, wherein in the aqueous zinc-nickel deposition bath the at least one brightening agent has a total concentration ranging from 20 mg/L to 650 mg/L, based on the total volume of the aqueous zinc-nickel deposition bath, preferably ranging from 50 mg/L to 600 mg/L, more preferably from 100 mg/L to 550 mg/L, most preferably from 150 mg/L to 500 mg/L.

However, in some cases, preferred is a method of the present invention, wherein in the aqueous zinc-nickel deposition bath the at least one brightening agent has a total concentration ranging from 700 mg/L to 1200 mg/L, based on the total volume of the aqueous zinc-nickel deposition bath, preferably ranging from 750 mg/L to 1100 mg/L, more preferably from 800 mg/L to 1000 mg/L, most preferably from 850 mg/L to 950 mg/L.

Preferred is a method of the present invention, wherein each of the at least one brightening agent comprises 4 to 25 carbon atoms, preferably 5 to 20 carbon atoms, more preferably 6 to 18 carbon atoms, even more preferably 7 to 15 carbon atoms, most preferably 8 to 13 carbon atoms. In some cases, very preferred are 10 to 14 carbon atoms.

Preferred is a method of the present invention, wherein the brightening agent source comprises a cationic brightening agent with a counter anion, a non-ionic brightening agent, and/or an inner salt brightening agent. Preferably, the inner salt brightening agent is a betaine brightening agent.

More preferred is a method of the present invention, wherein the at least one brightening agent is a compound (preferably one or more than one compound) selected from the group consisting of
- N-heteroaromatic compounds, preferably N-heteroaromatic compounds comprising at least one pyridine moiety or at least one pyridinium moiety;
- aldehydes, preferably aldehydes comprising at least one aromatic ring;
- ketones, preferably ketones comprising at least one aromatic ring; and
- sulfonic acids additionally comprising a mercapto group, a disulfide moiety, and/or a thioether moiety.

Preferred is a method of the present invention, wherein the N-heteroaromatic compounds (preferably as described above, or throughout the text, as being preferred) additionally comprise a benzyl group. Most preferably, the at least one brightening agent comprises at least one of such compounds (preferably as defined throughout the text as being preferred).

Preferred is a method of the present invention, wherein the N-heteroaromatic compounds (preferably as described before as being preferred) additionally (i.e. in addition to the N-heteroatom or preferably in addition to the benzyl group as defined above) comprise an amide group, a carboxylate group, a sulfonate group, and/or esters thereof. The amide group is preferably a carboxamide group.

Preferred is a method of the present invention, wherein the N-heteroaromatic compounds comprising at least one pyridinium moiety are selected from the group consisting of substituted pyridinium sulfobetaines, unsubstituted pyridinium sulfobetaines, substituted pyridiniumcarboxylates, unsubstituted pyridiniumcarboxylates, substituted pyridiniumcarboxamides, and unsubstituted pyridiniumcarboxamides.

Preferred substituted and unsubstituted pyridinium sulfobetaines comprise substituted and unsubstituted pyridinium alkyl sulfobetaines. A preferred unsubstituted pyridinium alkyl sulfobetaine is pyridinium propyl sulfobetaine (PPS).

Very preferred is a method of the present invention, wherein the N-heteroaromatic compounds comprising at least one pyridinium moiety are selected from the group consisting of N-benzylnicotinates and esters thereof, N-benzylnicotinamides, N-benzylpyridinum, N-benzylpyridiniumsulfonates and esters thereof, N-alkylnicotinates and esters thereof, and N-alkylnicotinamides. The carboxylate, amide, and sulfonate group, respectively, is preferably in ortho, meta, or para position of the pyridinium ring, most preferably in meta position. The benzyl group is attached to the ring nitrogen forming a quaternary ring nitrogen atom with a positive charge. In some cases, most preferred is a method of the present invention, wherein the at least one brightening agent comprises N-alkylnicotinates and esters thereof, and/or N-alkylnicotinamides. A very preferred N-alkylnico-tinate comprises 1-methylnicotinate (i.e. N-methylnicotinate), preferably including salts, esters, and amides thereof.

In some cases, preferred is a method of the present invention, wherein the N-heteroaromatic compounds comprising a pyridine moiety are selected from the group consisting of benzylpyridine, nicotinic acid and esters thereof, benzylnicotinate and esters thereof, and benzylnicotinamide. In these cases, the benzyl group is not attached to the ring nitrogen atom. Thus, the ring nitrogen preferably does not form a positive charge.

Preferred is a method of the present invention, wherein the at least one brightening agent comprises at least N-benzylnicotinates, esters thereof, N-benzylnicotinamides, N-alkylnicotinates, esters thereof, and/or N-alkylnicotinamides.

Very preferred is a method of the present invention, wherein the at least one brightening agent, most preferably the brightening agent source, comprises (preferably consists of) a compound of formula (la) and/or esters thereof, preferably of formula (lb) and/or esters thereof, 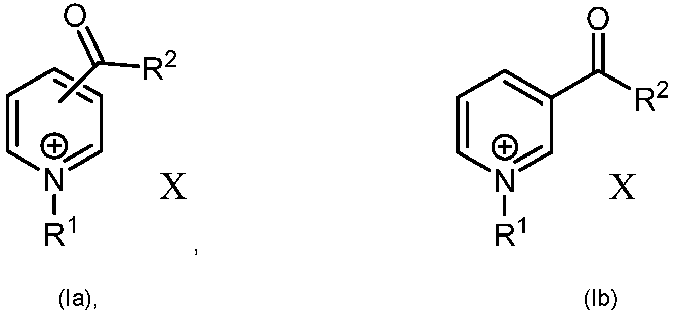 wherein
R¹ denotes a C1 to C4 alkyl, an unsubstituted phenyl alkylene, or a substituted phenyl alkylene,
R² denotes OH, OZ, OR³, NH₂, or NR³R⁴, wherein
   Z denotes an alkali metal cation, an ammonium cation, or an alkylammonium cation; preferably a sodium cation, a potassium cation, an ammonium cation, or a tetraalkylammonium cation;
   R³ independently denotes alkyl; preferably a C1 to C4 alkyl; and
   R⁴ independently denotes hydrogen or alkyl; preferably hydrogen or a C1 to C4 alkyl, and
   X denotes an anion selected from the group consisting of sulfate, methyl sulfate, formate, acetate, mesylate, tosylate, triflate, carbonate, and sulfonate.

In the context of the present invention, in R², in "OZ" "Z" denotes a moiety preferably compensating the negative charge of the carboxylate group. A preferred OZ comprises ONa and/or OK, wherein "Na" denotes sodium and "K" denotes potassium.

Preferably, in "NR³R⁴", R³ and R⁴ are either identical or different, preferably identical, i.e. both are alkyl, preferably both are a C1 to C4 alkyl.

In some cases, R¹ preferably denotes a substituted phenyl alkylene. This preferably means that the phenyl moiety comprises one or more than one substituent. Preferably, this one or more than one substituent is independently selected from the group consisting of carboxy, hydroxy, sulfonate, amino, C1 to C4 alkyl, C1 to C4 alkoxy, halogen, carbamoyl, cyano, esters, and salts thereof.

In compound of formula (la) the moiety -C(O)R² is in ortho, meta, or para position, preferably in meta position. Compound of formula (lb) is a compound of formula (la), wherein the moiety -C(O)R² is in meta position.

Preferred is a method of the present invention, wherein in R¹ the C1 to C4 alkyl (preferably as well the one or more than one substituent in the substituted phenyl alkylene defined above) individually comprises methyl, ethyl, n-propyl, iso-propyl, n-butyl, or iso-propyl; preferably methyl, ethyl, or iso-butyl, most preferably methyl. These preferred alkyl moieties also apply to the preferred C1 to C4 alkyl moieties of R³ and R⁴, respectively.

Preferred is a method of the present invention, wherein in R¹ the phenyl alkylene comprises benzyl.

Preferred is a method of the present invention, wherein R² denotes ONa, OK (i.e. M denotes a sodium or a potassium cation), or NH₂, preferably ONa (i.e. M denotes a sodium cation) or NH₂.

Preferred is a method of the present invention, wherein X comprises sulfate and/or methyl sulfate.

In X, sulfonate preferably comprises sulfonate (SO₃²⁻), methane sulfonate, methane disulfonate, and/or methane tri-sulfonate. Most preferred is a method of the present invention, wherein X comprises sulfate, methyl sulfate, and/or methane sulfonate, most preferably if R¹ comprises benzyl.

Even more preferred is a method of the present invention, wherein the at least one brightening agent, most preferably the brightening agent source, comprises a compound selected from the group consisting of compounds (A) to (D) 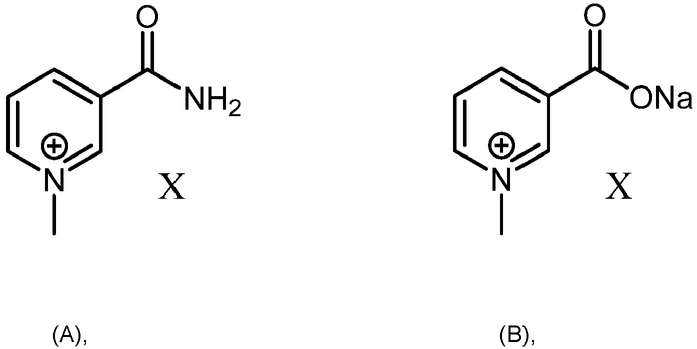 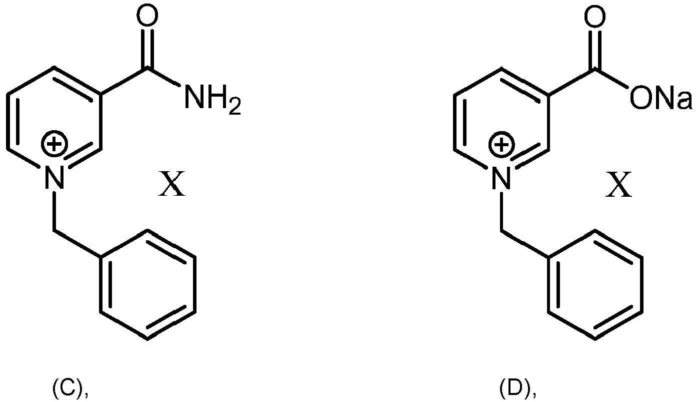 and esters thereof, wherein
X individually denotes an anion selected from the group consisting of sulfate, methyl sulfate, formate, acetate, mesylate, tosylate, triflate, carbonate, and sulfonate; preferably from the group consisting of sulfate and methyl sulfate.

Regarding X, most preferably all the aforementioned applies likewise individually to the compounds of formula (A), (B), (C), and (D).

Preferred is a method of the present invention, wherein the aldehydes comprising at least one aromatic ring comprise alkoxylated benzaldehydes, more preferably vanillin, piperonal, and/or anisaldehyde.

Preferred is a method of the present invention, wherein the ketones comprising at least one aromatic ring comprise benzalacetone and/or naphtylmethylketone.

Preferred is a method of the present invention, wherein the aqueous zinc-nickel deposition bath is substantially free of, preferably does not comprise, propane sultone, preferably is substantially free of, preferably does not comprise, any sultone.

### Complexing agents for nickel ions:

Preferred is a method of the present invention, wherein the aqueous zinc-nickel deposition bath further comprises one or more than one, preferably one, complexing agent for nickel ions. Complexing agents are needed in order to keep nickel ions in solution despite an alkaline pH environment. A use of only one complexing agent is preferred because the total concentration thereof can be better monitored and controlled. This is most preferred in a loop, preferably a closed-loop. However, although only one is preferred, this does not necessarily exclude the use of more than one complexing agent, which is basically possible.

A method of the present invention is preferred, wherein the one or more than one complexing agent for nickel ions comprises a chelating complexing agent, wherein preferably a chelating complexing agent is the only complexing agent for nickel ions in the aqueous zinc-nickel deposition bath. By using a chelating complexing agent, an even more efficient stabilization of the nickel ions in the aqueous zinc-nickel deposition bath is ensured. Most preferably, the one or more than one complexing agent for nickel ions is provided only once when initially setting up the aqueous zinc-nickel deposition bath, while substantially no additional complexing agent (neither of the same type nor a different complexing agent) is preferably added afterwards while the method of the present invention is carried out.

A method of the present invention is preferred, wherein the one or more than one complexing agent for nickel ions comprises an amine, preferably a diamine, more preferably an oligoamine, even more preferably a pentamine, most preferably the aqueous zinc-nickel deposition bath comprises tetraethylenepentamine (even most preferably only tetraethylenepentamine as complexing agent for nickel ions).

A method of the present invention is preferred, wherein the aforementioned amines, including the preferred amines, are the only complexing agents for nickel ions in the aqueous zinc-nickel deposition bath.

A preferred diamine comprises ethylenediamine.

A preferred oligoamine comprises diethylenetriamine, triethylenetetramine, and/or tetraethylenepentamine. In some cases, a method of the present invention is preferred, wherein the aqueous zinc-nickel deposition bath comprises one or more than one of aforementioned amines instead of tetraethylenepentamine. However, in other cases, a method of the present invention is preferred, wherein the aqueous zinc-nickel deposition bath comprises one or more of aforementioned amines in addition to tetraethylenepentamine.

Generally, a method of the present invention is preferred, wherein in the aqueous zinc-nickel deposition bath the one or more than one complexing agent for nickel ions comprises an amine having one or more than one, preferably two, primary amine group and one or more than one secondary amine group.

A method of the present invention is preferred, wherein the amine having one or more than one, preferably two, primary amine group and one or more than one secondary amine group, is the only complexing agent for nickel ions in the aqueous zinc-nickel deposition bath.

Furthermore, a method of the present invention is preferred, wherein the aqueous zinc-nickel deposition bath comprises only an initial concentration of the one or more than one complexing agent for nickel ions for at least one nickel ion turn over, more preferably for at least 2 nickel ion turn overs, even more preferably for at least 3 nickel ion turn overs, most preferably for a multitude of nickel ion turn overs, even most preferably for the entire life time of the aqueous zinc-nickel deposition bath utilized as catholyte.

A method of the present invention is preferred, wherein in the aqueous zinc-nickel deposition bath the zinc ions do not form a complex with the one or more than one complexing agent for nickel ions, preferably do not form a complex with an amine, more preferably do not form a complex with a diamine, even more preferably do not form a complex with an organic complexing agent. Most preferably, the zinc ions in the aqueous zinc-nickel deposition bath are strongly stable as hydroxo complexes such that no complex formation of zinc ions with the one or more than one complexing agent for nickel ions is observed under alkaline conditions.

### The anolyte:

The deposition compartment also comprises an anolyte. In the context of the present invention, the anolyte typically is an electrolyte being in direct contact with the at least on anode, wherein the catholyte is the aqueous zinc-nickel deposition bath being, at least partly, in direct contact with a cathode, i.e. with the substrate, preferably at least for the time the substrate is located in the deposition compartment.

A method of the present invention is preferred, wherein the anolyte is aqueous, preferably comprising water and sulfuric acid, most preferably comprising water and 5 vol.-% to 40 vol.-% sulfuric acid, based on the total volume of the anolyte.

### The at least one anode:

A method of the present invention is preferred, wherein in the deposition compartment the at least one anode is an insoluble anode, preferably an insoluble mixed metal oxide anode, more preferably an insoluble iridium oxide and/or tantalum oxide anode, most preferably an insoluble iridium/tantalum oxide on titanium anode.

### The at least one membrane:

In the deposition compartment said at least one anode and said at least one membrane is present, wherein the at least one membrane separates the anolyte from the catholyte. Most preferably the at least one membrane is a semi-permeable membrane. This means that the at least one membrane is selectively permeable. A method of the present invention is preferred, wherein the at least one membrane is a cation permeable membrane.

Said at least one membrane most preferably allows only for a diffusion of protons between the anolyte and catholyte, which ensures an efficient distribution of charges between the anolyte and the catholyte.

More preferred is a method of the present invention, wherein the at least one membrane comprises a fluorinated polymer, preferably polytetrafluoroethylene (PTFE) and/or (preferably or) perfluorosulfonic acid (PFSA).

Generally, the at least one membrane is not specifically limited in the context of the present invention, preferably as long as a sufficient separation between anolyte and catholyte is provided. Therefore, preferred is a method of the present invention, wherein the anolyte is separated from the catholyte by at least one separator, which most preferably is a membrane. In other cases, a ceramic is preferred as separator. Thus, throughout the present text, features related to the at least one membrane preferably also apply *mutatis mutandis* to the at least one separator.

A method of the present invention is preferred, wherein the at least one anode has a distance to the at least one membrane in a range from 0.5 mm to 5.0 mm, preferably from 0.75 mm to 4 mm, more preferably from 1.0 mm to 3.0 mm. This advantageously allows to keep the volume of the anolyte low, which in turn results in low amounts of wastewater from the anolyte.

A major advantage of the method of the present invention is that degradation products are not anodically formed due to the at least one membrane separating the anolyte from the catholyte. This means that neither the one or more than one complexing agent nor the at least one brightening agent is in direct contact with the at least one anode because they are restricted to the catholyte. Permeation of ions between catholyte and anolyte is only possible through the at least one membrane. This is a basic requirement for carrying out the method of the present invention in a loop, most preferably in a closed-loop, which most preferably constantly recycles an initial concentration of said one or more than one complexing agent for nickel ions. Most preferably, the at least one membrane substantially allows only permeation of hydrogen ions (formed in the anolyte) into the catholyte.

Thus, preferred is a method of the present invention, wherein the one or more than one complexing agent for nickel ions is not in contact with the at least one anode, most preferably is not in contact with any of the at least one anode. As a result, the concentration of the one or more than one complexing agent is preferably constant in the catholyte if the nickel ion source is carefully controlled, and no further complexing agents are added in an uncontrolled manner. Rather, it is preferably sufficient to provide only an initial concentration of the one or more than one complexing agent for nickel ions when setting up the aqueous zinc-nickel deposition bath, preferably the catholyte, wherein no additional complexing agent has to be added during the method of the present invention is carried out.

Preferred is a method of the present invention, wherein the at least one brightening agent is not in contact with the at least one anode, most preferably is not in contact with any of the at least one anode.

Most preferably, no organic compounds comprised in the aqueous zinc-nickel deposition bath (i.e. in the catholyte) are in contact with the at least one anode, most preferably are not in contact with any of the at least one anode.

Furthermore, preferably no cyanide and oxalate anions are anodically formed due to said separation.

### Step (c): contacting the substrate with the aqueous zinc-nickel deposition bath (i.e. the catholyte):

In step (c) of the method of the present invention, the substrate or the pre-rinsed substrate (if pre-rinsing is carried out) is contacted with the catholyte in the deposition compartment such that the zinc-nickel alloy is electrolytically deposited onto the substrate and thereby obtaining the zinc-nickel coated substrate.

During step (c), the at least one brightening agent present in the catholyte is typically partly consumed. Thus, after step (c) or after a step (c), meaning that the method of the present invention is repeated, the at least one brightening agent has a lower concentration than before step (c) or the respective previous step (c). In other words, after step (c), preferably after each step (c), the at least one brightening agent has a total concentration below the total concentration prior to step (c). To a certain extent this is acceptable and normal. This also means that not after each step (c) the at least one brightening agent is replenished. In other words, the at least one brightening agent has preferably a total concentration within a concentration range, wherein the concentration range has a lower limit, which is preferably not undercut during the method of the present invention.

Preferred is a method of the present invention, wherein in step (c) the temperature of the catholyte is ranging from 10°C to 50°C, preferably from 15°C to 45°C, more preferably from 17°C to 40°C, mot preferably from 20°C to 35°C, even most preferably from 22°C to 30°C.

Preferred is a method of the present invention, wherein in step (c) an electrical current is applied, preferably with a current density ranging from 0.3 A/dm² to 10 A/dm², more preferably from 0.4 A/dm² to 8 A/dm², even more preferably from 0.5 A/dm² to 7 A/dm².

More preferred is a method of the present invention, wherein in step (c) an electrical current is applied with a current density ranging from 0.3 A/dm² to 2 A/dm², more preferably from 0.4 A/dm² to 1.5 A/dm², even more preferably from 0.5 A/dm² to 1 A/dm². This most preferably applies to barrel applications.

More preferred is a method of the present invention, wherein in step (c) an electrical current is applied with a current density ranging from 0.5 A/dm² to 8 A/dm², more preferably from 1.1 A/dm² to 7 A/dm², even more preferably from 1.5 A/dm² to 6 A/dm². This most preferably applies to rack applications.

Preferred is a method of the present invention, wherein step (c) is carried out for a time ranging from 15 minutes to 180 minutes.

### Step (d): adding a brightening agent source to the catholyte:

In step (d) of the method of the present invention a brightening agent source is added to the catholyte, directly or indirectly. The brightening agent source is characterized in that it is substantially free of, preferably does not comprise, halogen anions. This is the essential feature in the context of the present invention. In particular in a loop, most preferably in a closed-loop, it is essential that no contaminations are incorporated and accumulated in such a loop over time (even in an almost closed loop).

In the context of the present invention, the term "loop" denotes a circulation without or at least without significant drag-out while the method of the present invention is carried out, preferably in combination with a wastewater recycling. It preferably denotes that compounds present in the aqueous zinc-nickel deposition bath as well as water circulate and are re-utilized without drag-out or at least without significant drag-out, most preferably with the proviso that rinse water is at least partly (preferably most of it) included in the circulation. Most preferably, the circulation includes a drain of excess water, most preferably by evaporation, which specifically allows to drain only water without losing relevant compounds. Such excess water is typically chemically formed during the method of the present invention is carried out.

Preferred is a method of the present invention, wherein the method is repeatedly carried out, preferably such that a loop is formed.

Step (d) is preferably a replenishing step for the at least one brightening agent. Thus, in step (d) "adding" is preferably a replenishing (and therefore preferably interchangeable with "adding").

The brightening agent source is added directly or indirectly. In the context of the present invention, the term "directly" denotes that the brightening agent source is added (directly) to the catholyte. This also means that the brightening agent source is added in the deposition compartment. The term "indirectly" denotes that the brightening agent source is separately pre-treated, preferably pre-dissolved, e.g. in a separate volume; and/or added into a separate compartment outside the deposition compartment and introduced into the catholyte in a subsequent step, most preferably in the mixing unit.

After step (d) the at least one brightening agent has a higher total concentration in the catholyte than prior to step (d).

Preferred is a method of the present invention, wherein the brightening agent source is replenishing the at least one brightening agent present in the catholyte, most preferably is only replenishing the at least one brightening agent present in the catholyte. Most preferably, this is the only brightening agent at all (or are the only brightening agents at all). Thus, preferably the at least one brightening agent remains identical in its chemical nature while the method of the present invention is carried out.

The brightening agent source is substantially free of, preferably does not comprise, halogen anions. This preferably means that the brightening agent source is substantially free of, preferably does not comprise, fluoride ions, chloride ions, bromide ions, and iodide ions. Most preferably, the brightening agent source is substantially free of, preferably does not comprise, at least chloride ions. Thus, an accumulation of detrimental halogen anions, preferably chloride, at least from this kind of source, is efficiently prevented.

### Step (e): rinsing the zinc-nickel coated substrate:

After step (c) or step (d), the zinc-nickel coated substrate is preferably rinsed with water in a rinsing compartment, such that a rinsed zinc-nickel coated substrate and rinse water is obtained. Preferably, the rinse water comprises at least a portion of said at least one brightening agent and/or (preferably and) of said one or more than one complexing agent for nickel ions, most preferably at least a portion of said at least one brightening agent and/or (preferably and) at least a portion of said one or more than one complexing agents for the nickel ions, both as defined above.

Thus, preferred is a method of the present invention, further comprising step
(e) rinsing the zinc-nickel coated substrate with water in a rinsing compartment, such that a rinsed zinc-nickel coated substrate and rinse water is obtained, wherein the rinse water comprises at least a portion of said at least one brightening agent and/or one or more than one complexing agent for the nickel ions (preferably complexing agents as defined throughout the text, preferably as being preferred).

A method of the present invention is preferred, wherein the rinsing compartment comprises two to five fluidically connected rinsing sub-compartments forming a rinsing cascade.

Such a rinsing cascade is particularly efficient in rinsing, since the concentration of zinc and nickel ions as well as of complexing agents and brightening agents rinsed off/washed away from the zinc-nickel coated substrate is effectively reduced stepwise, such that the most downstream rinsing sub-compartment comprises a significantly low total concentration thereof compared to the most upstream rinsing sub-compartment of the rinsing cascade, which comprises most thereof.

As already repeatedly mentioned above, it is very preferred that rinse water is not discarded but recycled and, at least partly, re-utilized. For that, the rinse water is preferably further treated. A method of the present invention is preferred, wherein the rinse water obtained from one or more than one rinsing sub-compartment is further treated (preferably as defined throughout the present text), at least from the most upstream rinsing sub-compartment.

Typically, the rinse water comprises zinc ions, nickel ions, at least a portion of said at least one brightening agent, and/or (preferably and) at least a portion of said one or more than one complexing agent for nickel ions. Typically, the individual concentration thereof is significantly lower than in the catholyte. However, in a loop, these compounds and ions, respectively, are preferably recycled and re-utilized. A respective treatment is preferably carried out in a respective treatment compartment.

### The first treatment compartment:

Preferred is a method of the present invention, wherein
at least a portion of the catholyte, at least a portion of the rinse water, and/or at least a portion of the pre-rinse water is treated in a first treatment compartment such that water is separated therefrom, resulting in separated water and a concentrated aqueous solution,
wherein, directly or indirectly, at least a portion of the concentrated aqueous solution is returned into the catholyte.

More preferably, at least a portion of the catholyte and at least a portion of the rinse water is treated in the first treatment compartment, most preferably at least a portion of the catholyte and (all or almost all of) the rinse water. If in the first treatment compartment portions from different sources are to be treated, they are preferably pooled together and treated as a combined portion.

Preferred is a method of the present invention, wherein the majority of the catholyte, the rinse water, and/or the pre-rinse water is treated in the first treatment compartment, preferably individually 80 vol.-% or more, more preferably 85 vol.-% or more, even more preferably 90 vol.-% or more, yet even more preferably 95 vol.-% or more, most preferably 99 vol.-% or more, even most preferably 99.9 vol.-% or more. In a most preferred case, all of the catholyte, the rinse water, and the pre-rinse water is treated in the first treatment compartment. Most preferably this applies to the majority of the catholyte and the rinse water.

As a result of this treatment, separated water and a concentrated aqueous solution is formed. This means that in this step preferably a splitting into two fractions is obtained. It does not mean that water is completely separated from all other compounds. It preferably means that at least some water is separated. The term "separated therefrom" means that H₂O in a chemical sense is separated from all other ingredients, i.e. in a sense of extracting a certain amount of H₂O therefrom.

The obtained separated water is very preferred because during the method of the present invention, water is typically introduced into the catholyte, e.g. by means of the nickel ion source for replenishing nickel ions. During the method, water is also chemically formed in the catholyte (protons are formed anodically which are migrating into the alkaline catholyte). However, in the first treatment compartment excessive water is separated and subsequently removed from the method of the present invention such that a basically constant volume of the catholyte is maintained over time. If such excessive water cannot be used in the method of the present invention (e.g. for rinsing), it is preferably disposed. This is preferably possible because this water is preferably substantially free of, more preferably does not comprise, nickel ions. The same preferably applies to zinc ions, complexing agents, and brightening agents. Thus, this water can be typically disposed without any further wastewater treatment. This is a very preferred benefit of the method of the present invention; the separated water is preferably excellently pure. Alternatively, this water is preferably used in other industrial methods.

Thus, a method of the present invention is preferred, wherein at least a portion of the separated water obtained in the first treatment compartment is disposed, wherein this portion comprises nickel ions in a concentration ranging from 0 mg/L to 1.0 mg/L, based on the total volume of the disposed portion, preferably from 0 mg/L to 0.5 mg/L, more preferably from 0.01 mg/L to 0.11 mg/L, and most preferably from 0.01 mg/L to 0.1 mg/L.

This is typically considered as being very pure regarding nickel ions. Furthermore, this is generally a feature of the separated water.

A method of the present invention is preferred, wherein this portion comprises zinc ions in a concentration ranging from 0 mg/L to 1.0 mg/L, based on the total volume of the disposed portion, preferably from 0 mg/L to 0.5 mg/L, more preferably from 0.01 mg/L to 0.11 mg/L, and most preferably from 0.01 mg/L to 0.1 mg/L. Also, this is typically considered as being very pure regarding zinc ions and is also a general feature of the separated water.

Preferably, only a pH adaptation is required for the water to be disposed.

Thus, the treatment in the first treatment compartment is a separation between water and the ingredients typically present in the aqueous zinc-nickel deposition bath. In other words, water is separated from nickel ions, zinc ions, at least one brightening agent, and one or more than one complexing agent for nickel ions.

Preferably, at least a portion of the separated water can be re-utilized in the method of the present invention. Thus, preferred is a method of the present invention, wherein at least a portion of the separated water is utilized in step (a-1) and/or step (e) as the water. This means that at least a portion of the separated water is utilized in the pre-rinsing compartment and/or the rinsing compartment, respectively. More preferred is a method of the present invention, wherein at least a portion of the separated water obtained in the first treatment compartment is returned into the rinsing compartment, more preferably into at least one of the rinsing sub-compartments of the rinsing cascade. This dramatically safes freshwater usage for rinsing.

As mentioned already above, a water removal is preferably needed in order to maintain a constant volume of the catholyte. Thus, a method of the present invention is preferred, wherein the catholyte basically has a constant volume during carrying out the method of the present invention, most preferably basically has a constant volume during carrying out the method of the present invention with the proviso that the basically constant volume is maintained without significant drag out. This is very preferred in a loop, most preferably in a closed-loop. For that reason, preferably at least a portion of at least the catholyte is treated in the first treatment compartment.

In contrast to the separated water, the concentrated aqueous solution comprises various compounds and ions, respectively, of the catholyte.

The concentrated aqueous solution preferably comprises zinc ions, nickel ions, at least one brightening agent, and/or (preferably and) one or more than one complexing agent, most preferably all individually as defined for the catholyte.

In order to perform the separation, a method of the present invention is preferred, wherein the first treatment compartment comprises a separator unit.

More preferred is a method of the present invention, wherein the first treatment compartment comprises an evaporator, preferably a vacuum evaporator. These are preferred means for the separator unit.

A method of the present invention is preferred, wherein in the vacuum evaporator a vacuum is applied in a range from 1 mbar to 100 mbar, preferably from 5 mbar to 70 mbar, more preferably from 10 mbar to 50 mbar, most preferably from 15 mbar to 35 mbar.

A method of the present invention is preferred, wherein in the first treatment compartment, preferably in the separator unit, more preferably in the evaporator, most preferably in the vacuum evaporator, water is separated at a temperature in a range from 18°C to 50°C, preferably from 23°C to 46°C, more preferably from 28°C to 42°C, most preferably from 31°C to 40°C.

By using the evaporator, preferably the vacuum evaporator, an efficient evaporation of the water can be achieved, in particular by reducing the atmospheric pressure, thereby allowing an efficient separation of water from nickel ions, the one or more than one complexing agent, and the at least one brightening agent.

By operating the vacuum evaporator at a temperature between 18°C and 50°C, an undesired heating or even thermal degradation of organic compounds, specifically of the one or more than one complexing agent as well as the at least one brightening agent is prevented.

A method of the present invention is preferred, wherein the vacuum evaporator is operated and controlled based on a density measurement of the concentrated aqueous solution, preferably the density of the concentrated aqueous solution is in a range from 1.08 kg/L to 1.30 kg/L, based on the total volume of the concentrated aqueous solution, more preferably from 1.10 kg/L to 1.26 kg/L, even more preferably from 1.15 kg/L to 1.24 kg/L, most preferably from 1.20 kg/L to 1.23 kg/L. A control based on density is excellently suited to operate the first treatment compartment, preferably the evaporator, most preferably the vacuum evaporator, automatically. The above-mentioned density ranges are most preferred. However, in some case a higher maximum density is acceptable as long as the concentrated aqueous solution does not form a phase separation. This possibly incudes maximum densities of e.g. 1.28 kg/L, 1.30 kg/L, in some cases even 1.32 kg/L. A phase separation also typically depends on the concentration of e.g. sulfate ions, carbonate ions, and hydroxide ions (e.g. of sodium and/or potassium), which may vary over time.

As defined above, the concentrated aqueous solution is aqueous. Thus, a method of the present invention is preferred, wherein the concentrated aqueous solution is homogeneous. This preferably means that the concentrated aqueous solution forms only a single phase; in other words, the concentrated aqueous solution preferably does not form a phase separation. Most preferably the concentrated aqueous solution does not comprise an organic phase separated from an aqueous phase.

Thus, even more preferred is a method of the present invention, wherein the concentrated aqueous solution is completely aqueous. This means that water is the only solvent.

By not exceeding the above-mentioned preferred maximum density of most preferably 1.26 kg/L (or even 1.28 kg/L or 1.30 kg/L or 1.32 kg/L), a phase separation is typically avoided in the context of the method of the present invention.

Preferably, the concentrated aqueous solution is returned directly or indirectly into the catholyte, preferably indirectly via the mixing unit. At least a portion of the concentrated aqueous solution is returned into the catholyte, preferably the majority thereof, most preferably the concentrated aqueous solution is entirely returned. Preferably, the concentrated aqueous solution is returned either continually or stepwise.

### The second treatment compartment:

As mentioned above, a preferred nickel ion source comprises sulfate ions. Since nickel ions are consumed in the electrolytically deposited zinc-nickel alloy but not the sulfate ions, sulfate ions typically accumulate over time. Furthermore, the catholyte typically has the tendency to form and accumulate carbonate anions. Both anions are usually well soluble in the catholyte. Although a certain concentration can be tolerated, over-accumulation of such anions is to be avoided.

Thus, a method of the present invention is preferred, further comprising step
(f) treating at least a portion of the catholyte in a second treatment compartment such that dissolved anions are separated from the catholyte, preferably by precipitation and/or ion exchange, most preferably by precipitation.

Preferred is a method of the present invention, wherein the dissolved anions at least comprise sulfate ions, alkyl sulfonate ions (preferably methane sulfonate ions), and/or alkyl sulfate ions (preferably methyl sulfate ions). The term "dissolved anions" refers to anions which are dissolved in the catholyte prior to step (f), i.e. without the treatment in step (f). This means that step (f) allows for at least a partial removal of anions which otherwise would remain dissolved in the catholyte and therefore accumulate.

By applying step (f) the concentration of the dissolved anions, most preferably of sulfate ions and/or (preferably and) carbonate ions, in the catholyte is significantly reduced and over-accumulation is avoided. As a result, the method of the present invention can be operated for extremely long time. Preferably, step (f) is applied when the dissolved anions have reached an undesired concentration, either individually or in total. Preferably, step (f) comprises a precipitation to remove one or more than one of such dissolved anions from the catholyte, most preferably by reducing the temperature of at least a portion of the catholyte in the second treatment compartment and thereby lowering the solubility of respective salts. Most preferably, the treating in step (f) results in the formation of a solid precipitate, preferably a sodium-containing solid precipitate, most preferably comprising at least sodium sulfate or sodium sulfate in addition to sodium carbonate.

A method of the present invention is preferred, wherein the precipitation is carried out at a temperature in a range from -5°C to 11°C, preferably in a range from -4°C to 10°C, more preferably in a range from -3°C to 8°C, even more preferably in a range from -2°C to 6°C, most preferably in a range from -1°C to 4°C.

Thus, a method of the present invention is most preferred, wherein the dissolved anions comprise at least sulfate ions, and wherein sulfate ions are preferably separated from the catholyte by precipitated sodium sulfate. This preferably also allows to remove sodium ions, which are typically present in large amounts in the catholyte.

### Halogen anions in the aqueous zinc-nickel deposition bath/catholvte:

As mentioned above, in step (d) a brightening agent source is added directly or indirectly to the catholyte, said source being substantially free of, preferably not comprising, halogen anions. It is highly preferred, to maintain a comparatively low halogen anion concentration in the entire catholyte over the entire lifetime of the catholyte. Most preferably, no halogen anions are present.

Preferred is a method of the present invention, wherein the catholyte (i.e. preferably likewise the aqueous zinc-nickel deposition bath) comprises halogen anions in a total concentration ranging from 0 g/L to 10 g/L, based on the total volume of the catholyte, preferably ranging from 0 g/L to 8 g/L, more preferably ranging from 0 g/L to 6 g/L, even more preferably ranging from 0 g/L to 5 g/L, most preferably ranging from 0 g/L to 4 g/L, even most preferably ranging from 0 g/L to 3 g/L. This preferably applies for at least one nickel ion turn over, more preferably for at least 2 nickel ion turn overs, even more preferably for at least 3 nickel ion turn overs, most preferably for a multitude of nickel ion turn overs, even most preferably for the entire lifetime of the aqueous zinc-nickel deposition bath utilized as catholyte.

Preferred is a method of the present invention, with the proviso, in addition to above-mentioned total concentration for halogen anions that the catholyte (i.e. preferably likewise the aqueous zinc-nickel deposition bath) comprises chloride ions in a total concentration ranging from 0 g/L to 3 g/L, more preferably from 0 g/L to 2.5 g/L, even more preferably from 0 g/L to 2 g/L, most preferably from 0 g/L to 1 g/L.

In some cases, a method of the present invention is preferred, wherein the lower concentration limit of the total concentration of halogen anions is not necessarily zero, but preferably 1 g/L, more preferably 0.5 g/L, most preferably 0.1 g/L. In many cases, such a low but undisturbing residual amount of halogen anions is not problematic.

It was already mentioned above that the catholyte might comprise further compounds and ions, respectively. Preferred is a method of the present invention, wherein the catholyte (i.e. preferably likewise the aqueous zinc-nickel deposition bath) further comprises (i.e. in addition to (i), (ii), (iii), and preferably the one or more than one complexing agent)
(iv) at least one kind of alkali metal cation,
(v) sulfate ions,
(vi) carbonate ions, and
(vii) halogen anions,
with the proviso that, based on the total volume of the catholyte,
- (i), (iii), and (iv) to (vii) together have a total concentration ranging from 20 g/L to 270 g/L, and
- the halogen anions do not exceed a total concentration of 10 g/L.

The aforementioned regarding the halogen anions, applies preferably likewise here.

This total concentration is purposely defined as a range, reflecting a condition directly after a step (f) is carried out, such as the lower end of 20 g/L, and directly prior to a step (f), such as the upper end of 270 g/L. In other words, a method of the present invention is preferred, wherein said total concentration is permanently maintained within this range while the method of the present invention is carried out. This preferably includes that the method of the present invention is repeated.

As already indicated above, a major contributor to said total concentration are sulfate ions, carbonate ions, and the at least one kind of alkali metal cation (preferably sodium ions and/or potassium ions; however, primarily sodium ions). If said total concentration reaches said 270 g/L, step (f) is most preferably to be carried out. However, step (f) can be carried out even at lower total concentrations. However, if said total concentration is significantly exceeded 270 g/L, the deposition quality is in some cases already significantly disturbed.

On the other hand, typically a total concentration of 20 g/L or slightly above is very desired throughout the method of the present invention. This is most preferably achieved after step (f) is carried out.

Thus, preferred is a method of the present invention, wherein (i), (iii), and (iv) to (vii) together have a total concentration ranging from 25 g/L to 260 g/L, preferably from 30 g/L to 250 g/L, more preferably from 35 g/L to 240 g/L, even more preferably from 40 g/L to 230 g/L, most preferably from 45 g/L to 220 g/L. Also, in these preferred ranges, the lower limits preferably apply to a condition directly after a step (f) is carried out, wherein the upper limits preferably apply to a condition directly prior to a step (f).

In some cases, preferred is a method of the present invention, wherein (i), (iii), and (iv) to (vii) together have a total concentration ranging from 180 g/L to 270 g/L, preferably from 190 g/L to 250 g/L, more preferably from 200 g/L to 230 g/L, most preferably from 210 g/L to 220 g/L. This most preferably applies directly before a step (f) is carried out. These are typical and preferred total concentrations triggering step (f).

In some cases, preferred is a method of the present invention, wherein (i), (iii), and (iv) to (vii) together have a total concentration ranging from 20 g/L to 100 g/L, preferably from 25 g/L to 85 g/L, more preferably from 30 g/L to 73 g/L, even more preferably from 35 g/L to 65 g/L, most preferably from 40 g/L to 60 g/L. This most preferably applies directly after a step (f) is carried out.

Preferred is a method of the present invention, wherein (v) and (vi) together have a total concentration of 20 g/L or more, preferably of 25 g/L or more, most preferably of 30 g/L or more. Such a total concentration of (v) and (vi) together is typically well acceptable and very preferred, in particular if an upper limit of 60 g/L is not exceeded. It is therefore very preferred to maintain such a total concentration during the entire method of the present invention for (v) and (vi) together.

In some cases, preferred is a method of the present invention, wherein (v) alone has a concentration ranging from 15 g/L to 50 g/L, preferably from 20 g/L to 45 g/L. Most preferably such a concentration is maintained during the entire method of the present invention. Most preferably, the lower limits apply directly after a step (f) is carried out, wherein the upper limits preferably apply directly prior to a step (f).

Summarizing, the method of the present invention allows for an economic, sustainable, continuous operation over an extended period of time, i.e. for several months, weeks, or even years. During the extended period of time, no nickel contaminated wastewater is produced and no valuable metal ions as well as complexing agents and brightening agents are lost due to drag out. Basically, only the amount of consumed nickel ions, zinc ions, and brightening agent has to be replenished. Furthermore, halogen anions are kept low such that no detrimental effects, such as corrosion in and on the equipment, are observed.

The present invention also refers to a specific aqueous zinc-nickel deposition bath for depositing a zinc-nickel alloy, the bath comprising
(i) nickel ions,
(ii) at least one brightening agent,
(iii) zinc ions, and
further comprising
(iv) at least one kind of alkali metal cation,
(v) sulfate ions,
(vi) carbonate ions, and
(vii) halogen anions,
with the proviso that, based on the total volume of the aqueous zinc-nickel deposition bath,
- (i), (iii), and (iv) to (vii) together have a total concentration ranging from 20 g/L to 270 g/L, and
- the halogen anions do not exceed a total concentration of 10 g/L.

The aforementioned regarding the method of the present invention, in particular regarding the aqueous zinc-nickel deposition bath and the catholyte, respectively, utilized in the method of the present invention, preferably likewise applies to the aqueous zinc-nickel deposition bath of the present invention (preferably if technically applicable). This most preferably applies to the aforementioned regarding the at least one brightening agent.

Most preferably, the aqueous zinc-nickel deposition bath of the present invention is not a freshly prepared aqueous zinc-nickel deposition bath but rather an "in-use" bath, i.e. an utilized aqueous zinc-nickel deposition bath. In other words, one that has been already contacted with an electrical current, preferably as defined in step (c) of the method of the present invention.

The present invention also refers to a specific brightening agent of formula (Ia) and/or esters thereof, preferably for zinc-nickel alloy deposition 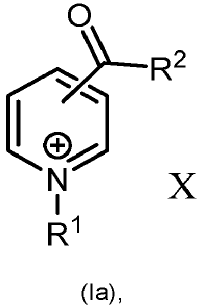 wherein
R¹ denotes a C1 to C4 alkyl, an unsubstituted phenyl alkylene, or a substituted phenyl alkylene,
R² denotes OH, OZ, OR³, NH₂, or NR³R⁴, wherein
   Z denotes an alkali metal cation, an ammonium cation, or an alkylammonium cation; preferably a sodium cation, a potassium cation, an ammonium cation, or a tetraalkylammonium cation;
   R³ independently denotes alkyl; preferably a C1 to C4 alkyl; and
   R⁴ independently denotes hydrogen or alkyl; preferably hydrogen or a C1 to C4 alkyl, and
X denotes an anion selected from the group consisting of sulfate, methyl sulfate, formate, acetate, mesylate, tosylate, triflate, carbonate, and sulfonate.

The aforementioned regarding the method of the present invention, in particular regarding the at least one brightening agent utilized in the aqueous zinc-nickel deposition bath and the catholyte, respectively, utilized in the method of the present invention, preferably applies likewise to the brightening agent of formula (Ia) of the present invention (preferably if technically applicable). This most preferably applies also to compounds of formula (lb), (A), (B), (C), (D), and esters thereof, as defined for the method of the present invention, which is likewise preferred for the brightening agent of the present invention.

Preferred is a brightening agent of the present invention, wherein in R¹ the C1 to C4 alkyl individually comprises methyl, ethyl, n-propyl, iso-propyl, n-butyl, or iso-propyl; preferably methyl, ethyl, or iso-butyl.

Preferred is a brightening agent of the present invention, wherein in R¹ the phenyl alkylene comprises benzyl.

Preferred is a brightening agent of the present invention, wherein R² denotes ONa, OK (i.e. Z denotes a sodium or potassium cation), or NH₂.

Preferred is a brightening agent of the present invention, wherein X comprises sulfate and/or methyl sulfate.

The present invention also refers to a use of a compound of formula (Ia) and/or esters thereof, as a brightening agent, 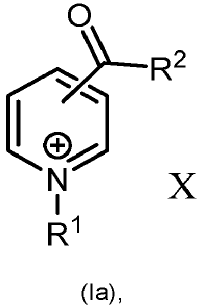 wherein
R¹ denotes a C1 to C4 alkyl, an unsubstituted phenyl alkylene, or a substituted phenyl alkylene,
R² denotes OH, OZ, OR³, NH₂, or NR³R⁴, wherein
   Z denotes an alkali metal cation, an ammonium cation, or an alkylammonium cation; preferably a sodium cation, a potassium cation, an ammonium cation, or a tetraalkylammonium cation;
   R³ independently denotes alkyl; preferably a C1 to C4 alkyl; and
   R⁴ independently denotes hydrogen or alkyl; preferably hydrogen or a C1 to C4 alkyl, and
X denotes an anion selected from the group consisting of sulfate, methyl sulfate, formate, acetate, mesylate, tosylate, triflate, carbonate, and sulfonate
for replenishing the brightening agent in an aqueous zinc-nickel deposition bath.

The aforementioned regarding the method of the present invention, in particular regarding the at least one brightening agent utilized in the aqueous zinc-nickel deposition bath and the catholyte, respectively, utilized in the method of the present invention, preferably applies likewise to the use of the present invention (preferably if technically applicable). This most preferably applies also to compounds of formula (lb), (A), (B), (C), (D), and esters thereof, as defined for the method of the present invention, which is likewise preferred for the use of the present invention.

Very preferred is a use of the present invention, wherein the compound of formula (Ia) and the esters thereof as defined throughout the present text, most preferably as defined throughout the present text as being preferred, is comprised in a brightening agent source or said source consists of said compound, with the proviso that said source is substantially free of, preferably does not comprise, halogen anions. The aforementioned regarding the brightening agent source in the context of the method of the present invention preferably also applies to the use of the present invention.

Preferred is a use of the present invention, wherein said compound of formula (Ia) and the esters thereof and the brightening agent in the aqueous zinc-nickel deposition bath (i.e. which is to be replenished) are identical.

Very preferred is a use of the present invention, wherein the compound of formula (Ia) and/or said esters thereof are used in a loop, preferably in a zinc-nickel deposition bath utilized in a method for depositing a zinc-nickel alloy according to the present invention.

The present invention is described in more detail by the following non-limiting examples.

### Examples

### (I) Brightening agent synthesis:

### Compound of formula (A):

In a 100 mL glass reactor with reflux condenser, 20 g (160 mmol) nicotinamide was dissolved in 50 ml water. To this solution 20.45 g (160 mmol) dimethyl sulfate was added at 25°C. The temperature was raised to 62°C. After completing the addition, the obtained reaction mixture was kept at 50°C for another 21 hours.

As a result, 90.4 g of a colorless solution were obtained (44 wt.-%; quantitative yield). NMR analysis was as follows:
¹H-NMR (D₂O; 400 MHz): δ 3.74 (s; 3H); 4.51 (s; 3H); 8.21 (t; ³J = 8 Hz); 8.92 (d; ³J = 4 Hz; 1 H); 9.00 (d; ³J = 4 Hz; 1 H); 9.30 (s; 1H).

### Compound of formula (C):

In a 50 mL glass reactor with reflux condenser, 5 g (40.1 mmol) nicotinamide was suspended in 15 mL acetonitrile at 25°C. Then 8.12 g (40.1 mmol) benzyl mesylate was added to this suspension and the reaction mixture was stirred for another 16 hours at 50°C. After the reaction was completed, the excess solvent was removed under vacuum.

As a result, 12.92 g (quantitative yield) of a yellow-orange oil was obtained. NMR analysis was as follows:
¹H-NMR (d6-DMSO, 400 MHz): δ 2.38 (s; 3H); 5.97 (s; 2H); 7.45 - 7.46 (m; 3H); 7.61 (dd; ³J = 4 Hz; 2H); 8.19 (s; 1 H); 8.28 - 8.32 (m; 1 H); 8.67 (s; 1 H); 9.01 (d; ³J = 4 Hz; 1H); 9.34 (d; ³J = 4 Hz; 1H); 9.66 (s; 1H).
¹³C-NMR (d6-DMSO; 100 MHz): δ 39.78; 63.61; 128.36; 129.04; 129.26; 134.19; 143.87; 144.82; 146.45; 148.55; 151.74; 162.74.

After synthesis, a further purification was obsolete because no detrimental halogen ions were present.

### (II) Brightening agent purification:

In an alternative approach, commercially available brightening agent of compound (D) with the only difference that X denotes chloride (purchased from BASF, Raschig) was purified via ion exchange column to replace chloride ions with sulfate ions.

### (III) Test deposition on substrates:

Test aqueous zinc-nickel deposition baths A, B, and C was set up as a catholyte in a deposition compartment (appr. 25 L) in order to deposit a zinc-nickel alloy on a load of small metal parts, i.e. screws M8x55. Test bath A was designed as comparative example, wherein B and C are baths according to the present invention, i.e. the brightening agent was modified in such a way that the respective brightening agent source did not contain significant amounts of halogen ions.

A, B, and C initially comprised about 1.3 g/L nickel (II) ions, about 8.5 g/L zinc (II) ions, and a diamine with additionally at least one secondary amine group as chelating complexing agent for the nickel ions. The pH was strongly alkaline about 12.5 and was adjusted with sodium hydroxide (initial about 120 to 140 g/L NaOH). The zinc (II) ions were present as hydroxo complexes.

As brightening agents the following compounds were used:
A: compound (D), wherein X denotes chloride;
B: compound (D), wherein X denotes sulfate (see above (II))
C: compound (C) wherein X denotes mesylate (see above (I))

The initial concentration of each brightening agent was about 200 mg/L, based on the total volume of the respective aqueous zinc-nickel deposition bath.

Subsequently, test plating was carried out until a bath utilization of 100 Ah/L was reached. Test plating took place with about 0.5 A/dm² at a temperature of about 25°C to 30°C for about 60 minutes for each load of screws. During this time, bath parameters as outlined in the following tables were monitored. Dummy plating was carried out with a steel sheet (20 cm x 30 cm). Typically, a detection limit for chloride in the present tests was about 50 mg/L and below. Nickel ions and brightening agent were replenished every 10 Ah (identical brightening agent); the nickel ion source (nickel sulfate) was free of any complexing agents and halogen ions. Zinc was replenished from metallic zinc dissolved under alkaline pH conditions. No additional complexing agent for zinc ions was used due to the formation of zinc hydroxide complexes under alkaline conditions. The catholyte and the anolyte were separated from each other by a cation exchange membrane. An insoluble iridium/tantalum oxide on titanium was used as anode. The distance between the anode and the respective membrane was below 5 mm. The anolyte, comprising water with sulfuric acid, is separated from the catholyte by said membrane such that neither the complexing agent nor the brightening agent was in contact with the anode.

Due to the separation between anolyte and catholyte, the catholyte does not comprise decomposition products such as cyanide and oxalate ions. This confirms that neither the complexing agent nor the at least one brightening agent was decomposed in the deposition compartment.

**Table 1; test aqueous zinc-nickel deposition bath A (comparative)**

| Ah/L | Zn [g/L] | Ni [g/L] | CI- [mg/L] | CO₃²⁻ [g/L] |
|---|---|---|---|---|
| 20 | 8.8 | 1.3 | < 50 | < 10 |
| 40 | 8.4 | 1.4 | < 50 | < 10 |
| 60 | 7.8 | 1.6 | ca. 700 | 10-20 |
| 80 | 7.9 | 1.4 | ca. 950 | 10-20 |
| 100 | 9.1 | 1.4 | ca. 1100 | 10-20 |

**Table 2; test aqueous zinc-nickel deposition bath B (inventive)**

| Ah/L | Zn [g/L] | Ni [g/L] | CI- [mg/L] | CO₃²- [g/L] |
|---|---|---|---|---|
| 20 | 9.1 | 1.3 | < 50 | < 10 |
| 40 | 8.6 | 1.4 | < 50 | < 10 |
| 70 | 8.2 | 1.5 | < 50 | < 10 |
| 80 | 7.5 | 1.4 | < 50 | < 10 |
| 100 | 7.3 | 1.5 | < 50 | < 10 |

**Table 3; test aqueous zinc-nickel deposition bath C (inventive)**

| Ah/L | Zn [g/L] | Ni [g/L] | Cl⁻ [mg/L] | CO₃²⁻ [g/L] |
|---|---|---|---|---|
| 20 | 8.5 | 1.5 | < 50 | 10-20 |
| 40 | 9.0 | 1.4 | < 50 | 10-20 |
| 60 | 8.5 | 1.2 | < 50 | 10-20 |
| 80 | 9.2 | 1.3 | < 50 | 10-20 |
| 100 | 8.7 | 1.5 | < 50 | 10-20 |

During the 100 Ah/L bath utilization, no treatment in the second treatment compartment was required, i.e. no critical concentration of sulfate ions and carbonate ions was yet reached.

The aforementioned test depositions clearly showed that with a brightening agent as used in B and C an increase of halogen ions was prevented because no other halogen ions than chloride ions were present. Thus, any increase of particularly chloride ions was impressively prevented. Furthermore, the entire method for depositing according to the present invention can be carried out on a comparatively low halogen ion concentration.

During the test plating, the nickel content in the zinc-nickel alloy was ranging from 13 to 14 %.

Generally, the method of the present invention can be carried out for barrel applications (as specifically shown above) as well as for rack applications (data not shown). Preferably for rack applications, generally a nickel concentration below 1 g/L is preferred, most preferably below 0.8 g/L. Furthermore, the substrates obtained after step (c) can be rinsed in a step (e), wherein the rinse water is further treated such that separated water and a concentrated aqueous solution is obtained (data also not shown).

## Claims

1. A method for depositing a zinc-nickel alloy on a substrate, the method comprising the steps:
(a) providing the substrate,
(a-1) optionally, pre-rinsing the substrate with water in a pre-rinsing compartment such that a pre-rinsed substrate and pre-rinse water is obtained,
(b) providing an aqueous zinc-nickel deposition bath as a catholyte in a deposition compartment, wherein
- the deposition compartment comprises at least one anode with an anolyte, and
- the anolyte is separated from the catholyte by at least one membrane, and the catholyte comprises
(i) nickel ions,
(ii) at least one brightening agent, and
(iii) zinc ions,
(c) contacting the substrate or the pre-rinsed substrate with the catholyte in the deposition compartment such that the zinc-nickel alloy is electrolytically deposited onto the substrate and thereby obtaining a zinc-nickel coated substrate, wherein
after step (c) the at least one brightening agent has a lower concentration than before step (c), **characterized in that** the method comprises after a step (c), step
(d) adding directly or indirectly a brightening agent source to the catholyte, said source being substantially free of, preferably not comprising, halogen anions.

2. The method of claim 1, wherein the at least one brightening agent is a compound selected from the group consisting of
- N-heteroaromatic compounds, preferably N-heteroaromatic compounds comprising at least one pyridine moiety or at least one pyridinium moiety;
- aldehydes, preferably aldehydes comprising at least one aromatic ring;
- ketones, preferably ketones comprising at least one aromatic ring; and
- sulfonic acids additionally comprising a mercapto group, a disulfide moiety, and/or a thioether moiety.

3. The method of claim 1 or 2, wherein the at least one brightening agent comprises at least N-benzylnicotinates, esters thereof, N-benzylnicotinamides, N-alkylnicotinates, esters thereof, and/or N-alkylnicotinamides.

4. The method of any of claims 1 to 3, further comprising step
(e) rinsing the zinc-nickel coated substrate with water in a rinsing compartment, such that a rinsed zinc-nickel coated substrate and rinse water is obtained, wherein the rinse water comprises at least a portion of said at least one brightening agent and/or one or more than one complexing agent for the nickel ions.

5. The method of any of claims 1 to 4, wherein
at least a portion of the catholyte, at least a portion of the rinse water, and/or at least a portion of the pre-rinse water is treated in a first treatment compartment such that water is separated therefrom, resulting in separated water and a concentrated aqueous solution,
wherein, directly or indirectly, at least a portion of the concentrated aqueous solution is returned into the catholyte.

6. The method of claim 5, wherein at least a portion of the separated water is utilized in step (a-1) and/or step (e) as the water.

7. The method of any of claims 1 to 6, wherein the at least one anode has a distance to the at least one membrane in a range from 0.5 mm to 5.0 mm, preferably from 0.75 mm to 4 mm, more preferably from 1.0 mm to 3.0 mm.

8. The method of any of claims 5 or 6, wherein the first treatment compartment comprises an evaporator, preferably a vacuum evaporator.

9. The method of any of claims 1 to 8, further comprising step
(f) treating at least a portion of the catholyte in a second treatment compartment such that dissolved anions are separated from the catholyte, preferably by precipitation and/or ion exchange, most preferably by precipitation.

10. The method of any of claims 1 to 9, wherein the catholyte further comprises
(iv) at least one kind of alkali metal cation,
(v) sulfate ions,
(vi) carbonate ions, and
(vii) halogen anions,
with the proviso that, based on the total volume of the catholyte,
- (i), (iii), and (iv) to (vii) together have a total concentration ranging from 20 g/L to 270 g/L, and
- the halogen anions do not exceed a total concentration of 10 g/L.

11. The method of claim 10, wherein (i), (iii), and (iv) to (vii) together have a total concentration ranging from 25 g/L to 260 g/L, preferably from 30 g/L to 250 g/L, more preferably from 35 g/L to 240 g/L, even more preferably from 40 g/L to 230 g/L, most preferably from 45 g/L to 220 g/L.

12. The method of any of claims 1 to 11, wherein the catholyte comprises halogen anions in a total concentration ranging from 0 g/L to 10 g/L, based on the total volume of the catholyte, preferably ranging from 0 g/L to 8 g/L, more preferably ranging from 0 g/L to 6 g/L, even more preferably ranging from 0 g/L to 5 g/L, most preferably ranging from 0 g/L to 4 g/L, even most preferably ranging from 0 g/L to 3 g/L.

13. An aqueous zinc-nickel deposition bath for depositing a zinc-nickel alloy, the bath comprising
(i) nickel ions,
(ii) at least one brightening agent,
(iii) zinc ions, and
further comprising
(iv) at least one kind of alkali metal cation,
(v) sulfate ions,
(vi) carbonate ions, and
(vii) halogen anions,
with the proviso that, based on the total volume of the aqueous zinc-nickel deposition bath,
- (i), (iii), and (iv) to (vii) together have a total concentration ranging from 20 g/L to 270 g/L, and
- the halogen anions do not exceed a total concentration of 10 g/L.

14. A brightening agent of formula (Ia) and/or esters thereof, 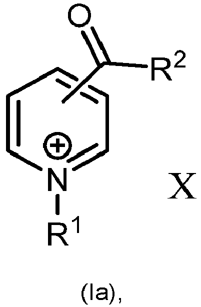 wherein
R¹ denotes a C1 to C4 alkyl, an unsubstituted phenyl alkylene, or a substituted phenyl alkylene,
R² denotes OH, OZ, OR³, NH₂, or NR³R⁴, wherein
Z denotes an alkali metal cation, an ammonium cation, or an alkylammonium cation; preferably a sodium cation, a potassium cation, an ammonium cation, or a tetraalkylammonium cation;
R³ independently denotes alkyl; preferably a C1 to C4 alkyl; and
R⁴ independently denotes hydrogen or alkyl; preferably hydrogen or a C1 to C4 alkyl, and
X denotes an anion selected from the group consisting of sulfate, methyl sulfate, formate, acetate, mesylate, tosylate, triflate, carbonate, and sulfonate.

15. Use of a compound of formula (Ia) and/or esters thereof, as a brightening agent, 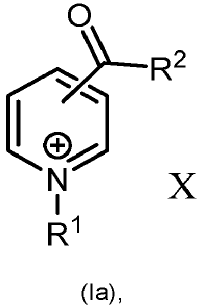 wherein
R¹ denotes a C1 to C4 alkyl, an unsubstituted phenyl alkylene, or a substituted phenyl alkylene,
R² denotes OH, OZ, OR³, NH₂, or NR³R⁴, wherein
Z denotes an alkali metal cation, an ammonium cation, or an alkylammonium cation; preferably a sodium cation, a potassium cation, an ammonium cation, or a tetraalkylammonium cation;
R³ independently denotes alkyl; preferably a C1 to C4 alkyl; and
R⁴ independently denotes hydrogen or alkyl; preferably hydrogen or a C1 to C4 alkyl, and
X denotes an anion selected from the group consisting of sulfate, methyl sulfate, formate, acetate, mesylate, tosylate, triflate, carbonate, and sulfonate
for replenishing the brightening agent in an aqueous zinc-nickel deposition bath.
